# FASCICULE DE BREVET EUROPEEN

(11) **EP 2 100 586 B1**
(45) Date de publication et mention de la délivrance du brevet: **22.09.2010**
(21) Numéro de dépôt: 09153860.3
(22) Date de dépôt: 27.02.2009
(51) Int. Cl.: A61K 8/368, A61K 8/37, A61K 8/90, A61Q 19/00

(54) **Composition cosmétique comprenant un composé d'acide ascorbique ou d'acide salicylique**
Kosmetische Zusammensetzung, die eine Ascorbinsäure- oder eine Salicylsäureverbindung enthält
Cosmetic composition comprising an ascorbic acid or salicylic acid compound

(30) Priorité: 11.03.2008 FR 0851556; 11.03.2008 FR 0851555
(43) Date de publication de la demande: 16.09.2009
(73) Titulaire: L'Oréal, 75008 Paris (FR)
(72) Inventeur: Monello, Aldo, 91600 Savigny sur Orge (FR)
(74) Mandataire: Kromer, Christophe

(56) Documents cités:
- EP-A- 1 415 645
- EP-A- 1 676 563
- WO-A-02/19984
- WO-A-2006/102004
- DE-A1- 10 356 895
- DE-A1- 19 935 763
- FR-A- 2 585 575
- US-A1- 2007 269 390

## Description

La présente invention a pour objet une émulsion huile-dans-eau cosmétique comprenant un actif choisi parmi un composé d'acide ascorbique ou un composé d'acide salicylique, et un mélange particulier de tensioactif.

L'acide ascorbique ou vitamine C, et ses dérivés, sont couramment utilisés du fait de leurs nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

Par ailleurs, il est connu d'utiliser l'acide salicylique et ses dérivés dans des compositions topiques, notamment cosmétiques ou dermatologiques, par exemple comme agent kéra-tolytique pour traiter l'acné ou comme agent d'antivieillissement. Les documents FR-A-2 581 542 et EP-A-378 936 décrivent de tels dérivés.

Mais l'utilisation de l'acide ascorbique ou de l'acide salicylique ou de leurs dérivés dans des émulsions, et en particulier dans des émulsions huile-dans-eau, notamment lorsqu'elles contiennent un système tensioactif comprenant un ester d'acide gras et de polyéthylène glycol , et un tensioactif additionnel choisi parmi les esters d'acide gras en C₁₆-C₂₂ et de sorbitan et les esters d'acide gras en C₁₆-C₂₂ et de glycéryle, a tendance à déstabiliser l'émulsion qui présente alors un déphasage d'huile en surface. Les globules d'huile dispersés dans la phase aqueuse ont un aspect grossier rendant l'émulsion non homogène.

Le document DE-A-10356895 décrit une émulsion huile-dans-eau contenant de l'acide ascorbique ou un de ses dérivés et un système émulsionnant comprenant du PEG-40-stéarate et du stéarate de glycéryle.

Le document EP-A-1676563 décrit une émulsion huile-dans-eau comprenant de l'acide ascorbique ou un de ses dérivés, un ester d'acide gras en C16-C22 et de sorbitan et un ester d'acide gras éthoxylé, qui est stabilisé par la présence d'un gélifiant hydrophile polysaccharide tel que la gomme de xanthane.

Or ce système tensioactif décrit précédemment est intéressant pour la texture particulière qu'il confère à ces émulsions. Ces dernières ont une texture épaisse et conviennent pour un conditionnement du produit cosmétique dans un pot (contrairement aux textures fluides qui sont conditionnées en tube ou en flacon pompe ; la texture épaisse est telle que la composition ne s'écoule pas instantanément en renversant le pot) ; une telle composition peut être prise facilement avec les doigts, s'étale bien sur la peau, de façon agréable, tout en pénétrant facilement ; elle confère de la douceur et ne présente pas d'effet collant.

Le but de la présente invention est donc de disposer d'une émulsion contenant un actif choisi parmi l'acide ascorbique, l'acide salicylique, l'un de leurs dérivés, et le système tensioactif décrit précédemment, qui soit stable, notamment pendant 24 heures à 55 °C, voire pendant 2 mois à 45 °C.

L'inventeur a découvert que la stabilité d'une telle émulsion peut être obtenue en ajoutant un polycondensat d'oxyde d'éthylène et d'oxyde de propylène.

De façon plus précise, l'invention a pour objet une composition sous forme d'émulsion huile-dans-eau comprenant :
- un composé d'acide ascorbique ou un composé d'acide salicylique ;
- un ester d'acide gras et de polyéthylène glycol ;
- un tensioactif additionnel choisi parmi les esters d'acide gras en C₁₆-C₂₂ et de sorbitan et les esters d'acide gras en C₁₆-C₂₂ et de glycéryle ;
- un polycondensat d'oxyde d'éthylène et d'oxyde de propylène.

Selon un premier mode de réalisation, l'invention a pour objet une composition sous forme d'émulsion huile-dans-eau comprenant :
- un composé d'acide ascorbique ;
- un ester d'acide gras et de polyéthylène glycol ;
- un tensioactif additionnel choisi parmi les esters d'acide gras en C₁₆-C₂₂ et de sorbitan et les esters d'acide gras en C₁₆-C₂₂ et de glycéryle ;
- un polycondensat d'oxyde d'éthylène et d'oxyde de propylène.

Selon un deuxième mode de réalisation, l'invention a pour objet une composition sous forme d'émulsion huile-dans-eau comprenant :
- un composé d'acide salicylique ;
- un ester d'acide gras et de polyéthylène glycol ;
- un tensioactif additionnel choisi parmi les esters d'acide gras en C₁₆-C₂₂ et de sorbitan et les esters d'acide gras en C₁₆-C₂₂ et de glycéryle ;
- un polycondensat d'oxyde d'éthylène et d'oxyde de propylène.

L'invention a également pour objet un procédé non thérapeutique de soin ou de maquillage des matières kératiniques comprenant l'application sur les matières kératiniques de la composition définie précédemment.

Le composé d'acide ascorbique présent dans la composition selon l'invention est avantageusement choisi parmi l'acide ascorbique et ses sels comme l'ascorbyl phosphate de magnésium, l'ascorbyl phosphate de sodium, et également l'acide ascorbique glycosylé, et leurs mélanges.

Le composé d'acide ascorbique est de préférence choisi parmi l'ascorbyl phosphate de magnésium et l'acide ascorbique glycosylé, et leur mélange.

Le composé d'acide ascorbique tel que décrit précédemment peut être présent dans l'émulsion selon l'invention en une teneur allant de 0,05 % à 10 % en poids, par rapport au poids total de la composition, de préférence allant de 0,05 % à 5 % en poids, et préférentiellement allant de 0,1 % à 3 % en poids.

Le composé d'acide salicylique présent dans la composition selon l'invention est avantageusement choisi parmi l'acide salicylique et les composés de formule (I) suivante : 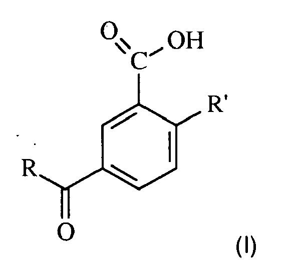 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone; lesdits groupes pouvant être substitués par un ou plusieurs substituants, identiques ou différents, choisis parmi (a) les atomes d'halogène (b) le groupe trifluorométhyle, (c) des groupes hydroxyle sous forme libre ou estérifiée par un acide ayant de 1 à 6 atomes de carbone ou (d) une fonction carboxyle sous forme libre ou estérifiée par un alcool inférieur ayant de 1 à 6 atomes de carbone ;
- R' est un groupe hydroxyle ;
- ainsi que leurs sels issus d'une base minérale ou organique.

De manière préférentielle , le radical R désigne une chaîne aliphatique saturée, linéaire, ramifiée ou cyclique contenant de 3 à 11 atomes de carbone ; une chaîne insaturée contenant de 3 à 17 atomes de carbone et comprenant une ou plusieurs doubles liaisons conjuguées ou non ; lesdites chaînes hydrocarbonées pouvant être substituées par un ou plusieurs substituants, identiques ou différents, choisis parmi (a) les atomes d'halogène (b) le groupe trifluorométhyle, (c) des groupes hydroxyle sous forme libre ou estérifiée par un acide ayant de 1 à 6 atomes de carbone ou (d) une fonction carboxyle sous forme libre ou estérifiée par un alcool inférieur ayant de 1 à 6 atomes de carbone ;
- ainsi que leurs sels obtenus par salification par une base minérale ou organique.

Les composés plus particulièrement préférés sont ceux dans lesquels le radical R est un groupement alkyle en C₃-C₁₁.

Parmi les composés de formule (I) particulièrement préférés, on peut citer :
l'acide n-octanoyl-5-salicylique (ou acide capryloyl salicylique) ; l'acide n-décanoyl -5-salicylique ; l'acide n-dodécanoyl-5-salicylique ; l'acide n-heptyloxy-5-salicylique et leurs sels correspondants.

Le composé d'acide salicylique est avantageusement choisi parmi l'acide salicylique et l'acide n-octanoyl-5-salicylique. On utilise plus particulièrement l'acide n-octanoyl-5-salicylique.

Les sels des composés de formule (I) peuvent être obtenus par salification par une base minérale ou organique. A titre d'exemple de base minérale, on peut citer les hydroxydes de métal alcalin ou alcalino-terreux comme l'hydroxyde de sodium, l'hydroxyde de potassium ou l'ammoniaque.

Parmi les bases organiques, on peut citer les amines et les alcanolamines. Les sels quaternaires comme ceux décrits dans le brevet FR 2 607 498 sont particulièrement intéressants.

Les composés de formule (I) utilisables selon l'invention sont décrits dans les brevets US 6,159,479 et US 5,558,871, FR 2,581,542, FR 2 607 498, US4,767,750, EP 378,936, US 5,267,407, US 5,667,789, US 5,580,549, EP-A-570,230.

Le composé d'acide salicylique tel que décrit précédemment peut être présent dans l'émulsion selon l'invention en une teneur allant de 0,05 % à 10 % en poids, par rapport au poids total de la composition, de préférence allant de 0,05 % à 5 % en poids, et préférentiellement allant de 0,1 % à 3 % en poids.

La composition selon l'invention comprend, à titre de tensioactif émulsionnant principal, au moins un ester d'acide gras et de polyéthylène glycol.

L'ester d'acide gras et de polyéthylène glycol présent dans la composition selon l'invention est de préférence un ester d'acides gras en C₁₆-C₂₂ comportant de 8 à 100 unités d'oxyde éthylène.

La chaîne grasse des esters peut être notamment choisie parmi les motifs stéaryle, béhényle, arachidyle, palmityle, cetyle et leurs mélanges tel que cétéaryle, et de préférence une chaîne stéaryle.

Le nombre d'unités d'oxyde d'éthylène peut aller de 8 à 100, de préférence de 10 à 80, et mieux de 10 à 50. Selon un mode particulier de réalisation de l'invention, ce nombre peut aller de 20 à 40.

A titre d'exemple d'ester d'acide gras et de polyéthylène glycol, on peut citer les esters d'acide stéarique comprenant respectivement 20, 30, 40, 50, 100 unités d'oxyde d'éthylène, tels que les produit commercialisés respectivement sous la dénomination Myrj 49 P (stéarate de polyéthylène glycol 20 OE ; nom CTFA : PEG-20 stearate), Myrj 51, Myrj 52 P (stéarate de polyéthylèneglycol 40 OE ; nom CTFA : PEG-40 stearate), Myrj 53, Myrj 59 P par la société CRODA.

L'ester d'acide gras et de polyéthylène glycol peut être présent dans la composition selon l'invention en une teneur allant de 0,1 % à 10 % en poids, par rapport au poids total de la composition, et de préférence allant de 0,1 % à 5 % en poids, et préférentiellement allant de 0,1 % à 3 % en poids.

La composition selon l'invention comprend également un tensioactif émulsionnant additionnel choisi parmi les esters d'acide gras en C₁₆-C₂₂ et de sorbitan et les esters d'acide gras en C₁₆-C₂₂ et de glycéryle.

Selon un premier mode de réalisation,de l'invention, la composition comprend un ester d'acide gras en C₁₆-C₂₂ et de sorbitan.

Les esters d'acide gras en C₁₆-C₂₂ et de sorbitan sont formés par estérification d'au moins un acide gras comportant au moins une chaîne alkyle linéaire saturée ou insaturée, ayant respectivement de 16 à 22 atomes de carbone, avec le sorbitol. Ces esters peuvent être notamment choisis parmi les stéarates, béhénates, arachidates, palmitates, oléates de sorbitan, et leurs mélanges. On utilise de préférence des stéarates et palmitates de sorbitan, et préférentiellement les stéarates de sorbitan.

L'ester d'acide gras en C₁₆-C₂₂ et de sorbitan présent dans la composition selon l'invention est avantageusement solide à une température inférieure ou égale à 45°C.

On peut citer à titre d'exemple d'ester de sorbitan utilisable dans composition selon l'invention, le monostéarate de sorbitan (nom CTFA : Sorbitan stearate) vendu par la société Croda sous la dénomination Span 60, le tristéarate de sorbitan vendu par la société Croda sous la dénomination Span 65 V, le monopalmitate de sorbitan (nom CTFA : Sorbitan palmitate) vendu par la société Croda sous la dénomination Span 40, le monoléate de sorbitan vendu par la société Croda sous la dénomination Span 80 V , le trioléate de sorbitan vendu par la société Uniquema sous la dénomination Span 85 V. de préférence, l'ester de sorbitan utilisé est le tristéarate de sorbitan.

L'ester d'acide gras en C₁₆-C₂₂ et de sorbitan peut être présent dans la composition selon l'invention en une teneur allant de 0,01 % à 10 % en poids, par rapport au poids total de la composition, de préférence allant de 0,01 % à 5 % en poids, et préférentiellement allant de 0,1 % à 3 % en poids.

L'ester de glycéryle et d'acide gras peut être obtenu notamment à partir d'un acide comportant une chaîne alkyle linéaire saturée, ayant de 16 à 22 atomes de carbone. Comme ester de glycéryle et d'acide gras, on peut citer notamment le stéarate de glycéryle (mono-, di- et/ou tri-stéarate de glycéryle) (nom CTFA : Glyceryl stearate), le ricinoléate de glycéryle, et leurs mélanges. De préférence, l'ester de glycéryle et d'acide gras utilisé est choisi parmi les stéarates de glycéryle.

L'ester de glycéryle et d'acide gras peut être présent en une quantité allant de 0,1 à 10 % en poids, par rapport au poids total de la composition, de préférence allant de 0,1 à 5 % en poids, et préférentiellement allant de 0,1 % à 3 % en poids.

La composition de l'invention peut être notamment un mélange de stéarate de glycéryle et de monostéarate de polyéthylène glycol 100 OE, et en particulier celui comprenant un mélange 50/50, commercialisé sous la dénomination Arlacel 165 par la société Croda.

La composition selon l'invention comprend un polycondensat d'oxyde d'éthylène et d'oxyde de propylène, et plus particulièrement un copolymère consistant en des blocs polyéthylène glycol et polypropylène glycol, comme par exemple les polycondensats tribloc polyéthylène glycol / polypropylène glycol / polyéthylène glycol. Ces polycondensats tribloc ont par exemple la structure chimique suivante :

H-(O-CH₂-CH₂)ₐ-(O-CH(CH₃)-CH₂)b-(O-CH₂-CH₂)ₐ-OH,

formule dans laquelle a va de 2 à 150, et b va de 1 à 100 ; de préférence a va de 10 à 130 et b va de 20 à 80.

Le polycondensat d'oxyde d'éthylène et d'oxyde de propylène a de préférence un poids moléculaire moyen en poids allant de 1000 à 15000, et de mieux allant de 1500 à 15000, et en particulier allant de 1500 à 10000, et encore mieux allant de 1500 à 5000.

Avantageusement, ledit polycondensat d'oxyde d'éthylène et d'oxyde de propylène a une température de trouble, à 10 g/l en eau distillée, supérieure ou égale à 20 °C, de préférence supérieure ou égale à 60 °C. La température de trouble est mesurée selon la norme ISO 1065.

Comme polycondensat d'oxyde d'éthylène et d'oxyde de propylène utilisable selon l'invention, on peut citer les polycondensats tribloc polyéthylène glycol / polypropylène glycol / polyéthylène glycol vendus sous les dénominations "SYNPERONIC" comme les « SYNPERONIC^{®} PE/F32 » (nom INCI : POLOXAMER 108), « SYNPERONIC^{®} PE/F108 » (nom INCI : POLOXAMER 338), "SYNPERONIC^{®} PE/ L44" (nom INCI : POLOXAMER 124) , « SYNPERONIC^{®} PE/L42 (nom INCI : POLOXAMER 122), "SYNPERONIC^{®} PE/F127 " (nom INCI : POLOXAMER 407), « SYNPERONIC^{®} PE/F88 » (nom INCI : POLOXAMER 238), « SYNPERONIC^{®} PE/L64 » (nom INCI : POLOXAMER 184) par la société CRODA , ou encore « LUTROL^{®} F68 » (nom INCI : POLOXAMER 188) par la société BASF.

Le polycondensat d'oxyde d'éthylène et d'oxyde de propylène peut être présent dans la composition selon l'invention en une teneur allant de 0,01 % à 5 % en poids, par rapport au poids total de la composition, de préférence allant de 0,05 % à 3 % en poids, et préférentiellement allant de 0,05 % à 1 % en poids.

La composition selon l'invention peut comprendre en outre un tensioactif anionique choisi parmi les sels alcalins de cétylphosphate. Les sels alcalins sont par exemple les sels de sodium, les sels de potassium. Le tensioactif ionique est de préférence le cétylphosphate de potassium.

On peut notamment utiliser le sel monopotassique de phosphate de monocétyle (nom INCI : potassium cetyl phosphate) vendu sous la dénomination « AMPHISOL K » par la société DSM Nutritional products.

Ce tensioactif anionique permet d'améliorer la stabilité de la composition à haute température (55 °C) pendant 2 mois.

Le tensioactif anionique peut être présent dans la composition selon l'invention en une teneur allant de 0,05 % à 5 % en poids, par rapport au poids total de la composition, de préférence allant de 0,5 à 3 % en poids, et préférentiellement allant de 0,1 % à 3 % en poids.

La composition selon l'invention peut comprendre un gélifiant hydrophile permettant d'épaissir la phase aqueuse de la composition.

Le gélifiant hydrophile peut être choisi par exemple parmi :
(i) les polymères carboxyvinyliques (comme les polymères d'acide acrylique, éventuellement réticulé), tels que les produits commercialisés sous les dénominations Carbopol (nom INCI : Carbomer) par la société Goodrich ;
(ii) les polyacrylamides et les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination « Hostacerin AMPS » (nom INCI : ammonium polyacryloyl-dimethyltaurate) ; les copolymères anioniques réticulés d'acrylamide et d'AMPS, se présentant sous la forme d'une émulsion, tels ceux commercialisés sous le nom de SEPIGEL 305 (nom C.T.F.A. : Polyacrylamide / C₁₃-₁₄ Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les copolymères anioniques réticulés d'acide acrylique et d'AMPS, se présentant sous la forme d'une émulsion, tels ceux commercialisés sous le nom de SIMULGEL EG (nom CTFA : Sodium acrylate/sodium acryloyldimethyltaurate copolymer / isohexadecane / Polysorbate 80) ; les copolymères acide 2-acrylamido 2-méthylpropane sulfonique /méthacrylate d'alcool en C12-C14 éthoxylé (ARISTOFLEX LNC de chez Clariant), les copolymères acide 2-acrylamido 2-méthylpropane sulfonique / méthacrylate de stéaryle éthoxylé (ARISTOFLEX HMS et ARISTOFLEX SNC de chez Clariant) ;
(iii) les polysaccharides comme les gommes de xanthane, les gomme de guar, les alginates, les polymères de celluloses comme l'hydroxyéthyl cellulose, l'hydroxypropyl cellulose, la carboxyméthyl cellulose ;
(iv) les composés inorganiques tels que les smectites, les hectorites modifiées ou non telles que les produits BENTONE commercialisés par la société Rheox, les produits LAPONITE commercialisés par la société Southern Clay Products, le produit VEEGUM HS commercialisé par la société R.T.Vanderbilt ; et leurs mélanges.

Parmi ces gélifiants hydrophiles, on choisira plus particulièrement les polysaccharides décrits précédemment et en particulier la gomme de xanthane.

Le gélifiant hydrophile peut être présent dans la composition selon l'invention en une teneur allant de 0,01 % à 10 % en poids, par rapport au poids total de la composition, de préférence allant de 0,1 % à 5 % en poids, et préférentiellement allant de 0,1 % à 3 % en poids.

La composition selon l'invention comprend une phase aqueuse.

La composition peut comprendre de l'eau en une teneur allant de 20 % à 95 % en poids, par rapport au poids total de la composition, de préférence allant de 30 % à 90 % en poids, et préférentiellement allant de 40 % à 70 % en poids.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition peut comprendre en outre un solvant organique miscible à l'eau à la température ambiante (25 °C) notamment choisi parmi les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbones, tels que la glycérine, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol ;
et leurs mélanges.

La composition selon l'invention peut comprendre un solvant organique miscible à l'eau à la température ambiante, notamment un polyol, en une teneur allant de 1 % à 20 % en poids, par rapport au poids total de la composition, et de préférence allant de 3 % à 15 % en poids.

Avantageusement, la composition selon l'invention a un pH allant de 3,0 à 8,0 , de préférence allant de 4,0 à 8,0, préférentiellement allant de 5,0 à 7,0, et plus préférentiellement allant de 5,5 à 6,5.

L'émulsion selon l'invention comprend également une phase huileuse.

Comme huiles plus particulièrement utilisables dans la composition de l'invention, on peut citer par exemple :
- les huiles hydrocarbonées d'origine animale, telles que le perhydrosqualène (ou squalane) ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;

- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom CTFA : Dicaprylyl ether) ; et les benzoates d'alcools gras en C₁₂-C₁₅ (Finsolv TN de FINETEX) ;
- leurs mélanges.

L'huile peut être présente dans la composition selon l'invention en une teneur allant de 1 % à 50 % en poids, par rapport au poids total de la composition, de préférence allant de 5 % à 40 % en poids, et préférentiellement allant de 5 % à 30 % en poids.

La phase huileuse de l'émulsion peut comprendre d'autres corps gras tels que les cires ; les gommes telles que les gommes de silicone (diméthiconol) ; les résines de silicone, et leurs mélanges.

La composition selon l'invention peut comprendre au moins un agent photoprotecteur organique actif dans l'UVA et/ou l'UVB (absorbeurs), hydrosolubles ou liposolubles ou bien insolubles dans les solvants cosmétiques couramment utilisés.

La composition selon l'invention présentant une bonne stabilité, elle est appropriée pour la formulation de filtres UV organiques : les filtres UV incorporés dans la composition ne sont pas dégradés en présence de composé d'acide ascorbique.

Les filtres organiques sont notamment choisis parmi les anthranilates ; les dérivés cinnamiques ; les dérivés de dibenzoylméthane ; les dérivés ascorbiques, les dérivés du camphre ; les dérivés de triazine autres que ceux de l'invention tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Avantageusement, on utilise un filtre protecteur organique non ionique.

L'agent photoprotecteur peut être présent dans la composition selon l'invention en une teneur allant de 0,01 % à 30% en poids, par rapport au poids total de la composition, de préférence allant de 0,1 à 25% en poids , et préférentiellement allant de 0,1 % à 20 % en poids.

La composition selon l'invention peut comprendre en outre des charges.

Par charges, il faut comprendre des particules de toute forme, incolores ou blanches, minérales ou organiques, insolubles dans le milieu de la composition quelle que soit la température à laquelle la composition est fabriquée, et qui ne colorent pas la composition.

Les charges peuvent être de toute forme, plaquettaires, sphériques ou oblongues, quelle que soit la forme cristallographique (par exemple feuillet, cubique, hexagonale, orthorombique, etc) . On peut citer : le talc, le mica, la silice, le kaolin, de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthanes, les charges composites, les microsphères de silice creuses, et les microcapsules de verre ou de céramique.

Les charges peuvent être présentes dans la composition en une teneur allant de 0,1 % à 15 % en poids, et de préférence allant de 0,1 % à 10 % en poids, et préférentiellement de 0,1 % à 5 % en poids, par rapport au poids total de la composition.

La composition selon l'invention peut comprendre en outre un actif choisi parmi les agents desquamants capables d'agir, soit en favorisant l'exfoliation, soit sur les enzymes impliquées dans la desquamation ou la dégradation des cornéodesmosomes, les agents hydratants, les agents dépigmentants ou pro-pigmentants, les agents anti-glycation, les inhibiteurs de NO-synthase, les inhibiteurs de 5α-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents anti-microbiens, les agents tenseurs, les agents anti-pollution ou anti-radicalaires, les agents anti-inflammatoires, les actifs lipolytiques ou ayant une activité favorable, directe ou indirecte, sur la diminution du tissu adipeux, les agents agissant sur la microcirculation, et les agents agissant sur le métabolisme énergétique des cellules.

Avantageusement, l'émulsion selon l'invention peut avoir une viscosité, mesurée à 25 °C, à une vitesse de cisaillement de 200 min⁻¹ (200 tours par minute, soit une fréquence de 50 Hz), allant de 1 à 4 Pa.s (10 à 40 poises), et de préférence allant de 2 à 3,5 Pa.s (20 à 35 poises). La viscosité est mesurée à 25 °C avec un viscosimètre RHEOMAT 180 de chez METTLER équipé d'un mobile n °3, la mesure étant effectuée après 10 minutes de rotation du mobile (temps au bout duquel on observe une stabilisation de la viscosité et de la vitesse de rotation du mobile), à un cisaillement de 200 min⁻¹.

La composition selon l'invention est en particulier destinée à un usage topique, notamment cosmétique ou dermatologique.

De façon connue, la composition cosmétique ou dermatologique de l'invention peut contenir également des adjuvants habituels dans le domaine cosmétique ou dermatologique, tels que les conservateurs, les parfums, les bactéricides, les absorbeurs d'odeur, les matières colorantes, les sels, les tensioactifs, les épaississants, les bases. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse.

La composition selon l'invention peut être appliquée sur la peau, les poils, les cils, les cheveux, les ongles ou les lèvres, suivant l'usage auquel elle est destinée. Elle peut ainsi être utilisée dans un procédé de traitement cosmétique de la peau, comprenant l'application de la composition selon l'invention sur la peau, par exemple en vue de la tonifier, de la régénérer, de lisser les ridules de la peau, et/ou pour lutter contre le vieillissement cutané, contre les méfaits des rayonnements UV et/ou pour renforcer les tissus cutanés contre les agressions de l'environnement.

En variante, la composition selon l'invention peut être utilisée pour la fabrication d'une préparation dermatologique.

La composition peut être une composition de soin, notamment être un produit de soin de la peau tels qu'une base de soin pour la peau, une crème de soin (crème de jour, de nuit, anti-rides), une base de maquillage ; une composition de soin pour les lèvres (baume à lèvres) ; une composition de protection solaire ou autobronzante.

La composition peut également être une composition de maquillage, notamment de maquillage de la peau, des lèvres, des cils, des sourcils, des cheveux. En particulier, la composition de maquillage peut être un fond de teint, un fard à joue, un fard à paupière, un produit anti-cernes, un produit de maquillage du corps.

Avantageusement, la composition est une composition non rincée.

L'émulsion selon l'invention peut être préparée selon le mode opératoire général suivant : Mélanger les constituants de la phase aqueuse en chauffant à une température d'environ 70 °C. Mélanger par ailleurs les huiles et les tensioactifs en chauffant à une température d'environ 80 °C. Verser la phase grasse dans la phase aqueuse, à une température d'environ 70 °C puis agiter pendant 10 minutes à l'aide d'une turbine, à grande vitesse. Refroidir l'émulsion obtenue à environ 60 °C. Ajouter ensuite les épaississants, puis agiter à nouveau pendant 10 minutes. Refroidir à environ 50 °C. Introduire ensuite l'acide ascorbique ou dérivé préalablement mélangé avec de l'eau, puis les autres actifs éventuellement.

L'invention sera maintenant illustrée à l'aide des exemples non limitatifs suivants.

### Exemple 1 et comparatif 1' :

On a préparé une composition de soin du visage sous forme d'émulsion huile-dans-eau selon l'invention (exemple 1) ayant la composition suivante :
- Mélange de monostéarate de glycéryle et de stéarate de polyéthylèneglycol (100 OE) (ARLACEL 165 FL de chez CRODA) 2,5 g
- Stéarate de PEG (20 OE) (MYRJ 49 P de chez Uniquema) 1 g
- Alcool stéarylique 1 g
- Acide stéarique 3 g
- Huile d'amande d'abricot 5 g
- Fraction liquide de beurre de karité 6 g
- Cyclohexasiloxane5 g
- Tetrapentanoate de pentaerythrityl 5 g
- Isohexadécane 3 g
- Cire d'abeille 0,75 g
- Cire de silicone (Dow Corning 2501 cosmetic wax) 1 g
- Mélange diméthiconol et dimethicone (Dow Corning 1503 Fluid) 1,5 g
- ascorbyl phosphate de magnésium 0,5 g
- acide ascorbique glycosylé 1 g
- Triéthanolamine 0,85 g
- copolymère acrytamide/acrylamido 2-méthyl propane sulfonate de sodium en émulsion inverse à 40% dans isohexadecane/eau (Simulgel 600 de chez Seppic) 1,4 g
- Charges 3,1 g
- Nacre 0,5 g
- Adénosine 0,1 g
- Cetyl phosphate de potassium 1,5 g
- Copolymère bloc OE-OP-OE (Synperonic^{®} PE/F108 de chez CRODA) 0,2 g
- Glycérol 8 g
- Conservateurs qs
- Eau qsp 100 g
On a également préparé une composition similaire mais ne contenant pas de Copolymère bloc OE-OP-OE (Synperonic^{®} PE/F108 de chez CRODA) et de cétyl phosphate de potassium (composition 1' ne faisant pas partie de l'invention) (quantités supprimées remplacées par le même poids d'eau).

L'émulsion de l'exemple 1 présente une bonne stabilité après 24 heures à 55 °C et également après 2 mois à 45 °C. En observation au microscope, l'émulsion obtenue est fine et serrée.

La composition est appliquée sur le visage en utilisation quotidienne de jour.

L'émulsion comparative de l'exemple 1' est instable après conservation pendant 2 mois à 45 °C : l'émulsion, observée au microscope, est plus grossière, plus dégradée. A l'oeil nu, on observe un déphasage avec relarguage en surface de la phase huileuse.

### Exemple 2 et comparatif 2' :

On a préparé une composition de soin du visage sous forme d'émulsion huile-dans-eau selon l'invention (exemple 2) ayant la composition suivante :
   - Mélange de monostéarate de glycéryle et de stéarate de polyéthylèneglycol (100 OE) (ARLACEL 165 FL de chez CRODA) 2,5 g
   - Stéarate de PEG (20 OE) (MYRJ 49 P de chez Uniquema) 1 g
   - Alcool stéarylique 1 g
   - Acide stéarique 3 g
   - Huile d'amande d'abricot 7,5 g
   - Huile de son de riz 4,5 g
   - Cire d'abeille 1,7 g
   - Cyclohexasiloxane 7 g
   - Mélange de poly dimethylsiloxane réticulé et de poly dimethylsiloxane (6 cst) (24/76) (KSG 16 de chez Shin Etsu) 2 g
   - ascorbyl phosphate de magnésium 0,5 g
   - acide ascorbique glycosylé 1 g
   - Triéthanolamine 0,80 g
   - copolymère acrylamide/acrylamido 2-méthyl propane sulfonate de sodium en émulsion inverse à 40% dans isohexadecane/eau (Simulgel 600 de chez Seppic) 1,3 g
   - Charges 3,1 g
   - Nacre 0,5 g
   - Adénosine 0,1 g
   - Cetyl phosphate de potassium 1,5 g
   - Copolymère bloc OE-OP-OE (Synperonic^{®} PE/F108 de chez CRODA) 0,2 g
   - Glycérol 5 g
   - Butylène glycol 2 g
   - Conservateurs qs
   - Eau qsp 100 g
   On a également préparé une composition similaire mais ne contenant pas de Copolymère bloc OE-OP-OE (Synperonic^{®} PE/F108 de chez CRODA) et de cétyl phosphate de potassium (composition 2' ne faisant pas partie de l'invention) (quantités supprimées remplacées par le même poids d'eau).

L'émulsion de l'exemple 2 présente une bonne stabilité après 24 heures à 55 °C et également après 2 mois à 45 °C. En observation au microscope, l'émulsion obtenue est fine et serrée.

La composition est appliquée sur le visage en utilisation quotidienne de jour.

L'émulsion comparative de l'exemple 2' est instable après conservation pendant 2 mois à 45 °C : l'émulsion, observée au microscope, est plus grossière, plus dégradée. A l'oeil nu, on observe un déphasage avec relarguage en surface de la phase huileuse.

### Exemple 3 :

On a préparé une composition de soin du visage sous forme d'émulsion huile-dans-eau ayant la composition suivante :
- Stéarate de glycéryle 2 g
- Stéarate de polyéthylèneglycol (40 OE) (MYRJ 52 P de chez CRODA) 2,5 g
- Tristéarate de sorbitan (SPAN 65 V de chez CRODA° 0,9 g
- Alcool cétylique 4,4 g
- Huile d'amande d'abricot 5 g
- Vaseline 2,5 g
- Cire d'abeille 0,75 g
- Cyclohexasiloxane 9 g
- Mélange poly dimethylsiloxane alpha-omega dihydroxyle / cyclopenta dimethylsiloxane (14.7/85.3) (DOW CORNING 1501 FL de chez Dow Corning) 4 g
- ascorbyl phosphate de magnésium 0,5 g
- acide ascorbique glycosylé 2 g
- Triéthanolamine 1,20 g
- Charges 3 g
- Adénosine 0,1 g
- Tocophérol 0,2 g
- Cetyl phosphate de potassium 1,5 g
- Copolymère bloc OE-OP-OE (Synperonic^{®} PE/F108 de chez CRODA) 0,2 g
- Glycérol 5 g
- Conservateurs qs
- Eau qsp 100 g

L'émulsion obtenue présente une bonne stabilité après 24 heures à 55 °C et également après 2 mois à 45 °C. En observation au microscope, l'émulsion obtenue est fine et serrée.

La composition est appliquée sur le visage en utilisation quotidienne de jour.

### Exemple 4 et comparatif 4' :

On a préparé une composition de soin du visage sous forme d'émulsion huile-dans-eau ayant la composition suivante :
- Stéarate de glycéryle 2 g
- Stéarate de polyéthylèneglycol (40 OE) (MYRJ 52 P de chez CRODA) 1,2 g
- Tristéarate de sorbitan (SPAN 65 V de chez CRODA) 0,9 g
- Alcool cétylique 4,4 g
- Huile d'amande d'abricot 5 g
- Vaseline 2,5 g
- Cire d'abeille 0,75 g
- Cyclohexasiloxane 9 g
- Mélange poly dimethylsiloxane alpha-omega dihydroxyle / cyclopenta dimethylsiloxane (14.7/85.3) (DOW CORNING 1501 FL de chez Dow Corning) 4 g
- ascorbyl phosphate de magnésium 0,5 g
- acide ascorbique glycosylé 2 g
- Triéthanolamine 1,20 g
- Amidon de maïs 3 g
- Adénosine 0,1 g
- Tocophérol 0,2 g
- Cetyl phosphate de potassium 1,5 g
- Copolymère bloc OE-OP-OE (Synperonic^{®} PE/F108 de chez CRODA) 0,4 g
- Glycérol 5 g
- Octane-1,2 diol 0,3 g
- Conservateurs qs
- Eau qsp 100 g
On a également préparé une composition similaire mais ne contenant pas de Copolymère bloc OE-OP-OE (Synperonic^{®} PE/F108 de chez CRODA) (composition 4' ne faisant pas partie de l'invention) (quantité supprimée remplacée par le même poids d'eau).
L'émulsion de l'exemple 4 présente une bonne stabilité après 24 heures à 55 °C. En observation au microscope, l'émulsion obtenue est fine et serrée.

La composition est appliquée sur le visage en utilisation quotidienne de jour.

L'émulsion comparative de l'exemple 4' est instable après conservation pendant 24 heures à 55 °C : l'émulsion, observée au microscope, est plus grossière, plus dégradée. A l'oeil nu, on observe un relarguage d'huile en surface.

### Exemple 5 :

On a préparé une composition de soin du visage sous forme d'émulsion huile-dans-eau ayant la composition suivante :
- Mélange de monostéarate de glycéryle et de stéarate de polyéthylèneglycol (100 OE) (ARLACEL 165 FL de chez CRODA) 2 g
- Stéarate de PEG (20 OE) (MYRJ 49 P de chez Uniquema) 0,8 g
- Alcool stéarylique 1 g
- Mélange de mono, distéarate (36/64) de glycéryle 3 g
- Acide stéarique 3 g
- 4-tertio-butyl-4'-méthoxy-dibenzoylméthane 3 g
- 2-cyano-3,3-diphénylacrylate de 2-éthyl hexyle 7 g
- Salicylate de 2-éthyl hexyle 5 g
- Octyl dodécanol 3 g
- Cyclopentasiloxane 10 g
- Cire d'abeille 1 g
- Myristate de myristyle 2 g
- acide n-octanoyl 5-salicylique 0,3 g
- Triéthanolamine 0,46 g
- Acide poly acrylamidométhyl propane sulfonique neutralisé partiellement à l'ammoniaque et hautement réticulé (Hostacerin AMPS de chez Clariant) 1,3 g
- Charges 3,2 g
- Nacre 1 g
- Adenosine 0,1 g
- Dispersion aqueuse de protéine de soja à 7 % MA 5 g
- Cetyl phosphate de potassium 1,5 g
- Copolymère bloc OE-OP-OE (Synperonic^{®} PE/F108 de chez CRODA) 0,2 g
- Glycérol 7 g
- Conservateurs qs
- Eau qsp 100 g

Cette émulsion présente une bonne stabilité après 24 heures à 55 °C et également après 2 mois à 45 °C. En observation au microscope, l'émulsion obtenue est fine et serrée.

La composition est appliquée sur le visage en utilisation quotidienne de jour.

On a préparé une composition de soin du visage sous forme d'émulsion huile-dans-eau ayant la composition suivante :
- Mélange de monostéarate de glycéryle et de stéarate de polyéthylèneglycol (100 OE) (ARLACEL 165 FL de chez CRODA) 2 g
- Stéarate de PEG (20 OE) (MYRJ 49 P de chez Uniquema) 0,8 g
- Alcool stéarylique 1 g
- Acide stéarique 3 g
- Sel disodique de l'acide éthylène diamine tétracétique 0,1 g
- 4-tertio-butyl-4'-méthoxy-dibenzoylméthane 3 g
- 2-cyano-3,3-diphénylacrylate de 2-éthyl hexyle 7 g
- Salicylate de 2-éthyl hexyle 5 g
- Octyl dodécanol 3 g
- Cyclopentasiloxane 10 g
- Cire d'abeille 1 g
- Myristate de myristyle 2 g
- acide n-octanoyl 5-salicylique 0,3 g
- Triéthanolamine 0,46 g
- Acide poly acrylamidométhyl propane sulfonique neutralisé partiellement à l'ammoniaque et hautement réticulé (Hostacerin AMPS de chez Clariant) 1,3 g
- Charges 3,2 g
- Nacre 1 g
- Adenosine 0,1 g
- Dispersion aqueuse de protéine de soja à 7 % MA 5 g
- Copolymère bloc OE-OP-OE (Synperonic^{®} PE/F108 de chez CRODA) 0,2 g
- Glycérol 7 g
- Conservateurs qs
- Eau qsp 100 g

On a également préparé une composition similaire mais ne contenant pas de Copolymère bloc OE-OP-OE (Synperonic^{®} PE/F108 de chez CRODA) (composition 6' ne faisant pas partie de l'invention) (quantité supprimée remplacée par le même poids d'eau).

L'émulsion de l'exemple 6 présente une bonne stabilité après 24 heures à 55 °C. En observation au microscope, l'émulsion obtenue est fine et serrée.

La composition est appliquée sur le visage en utilisation quotidienne de jour.

L'émulsion comparative de l'exemple 6' est instable après conservation pendant 24 heures à 55 °C : l'émulsion, observée au microscope, est plus grossière, plus dégradée. A l'oeil nu, on observe un relarguage d'huile en surface.

## Revendications

1. Composition sous forme d'émulsion huile-dans-eau comprenant :
- un ester d'acide gras et de polyéthylène glycol ;
- un tensioactif additionnel choisi parmi les esters d'acide gras en C₁₆-C₂₂ et de sorbitan et les esters d'acide gras en C₁₆-C₂₂ et de glycéryle ;
- un polycondensat d'oxyde d'éthylène et d'oxyde de propylène consistant en des blocs polyéthylène glycol et polypropylène glycol ;
- un actif choisi parmi l'acide ascorbique et ses sels , l'acide ascorbique glycosylé, l'acide salicylique et les dérivés d'acide salicylique de formule (I) : 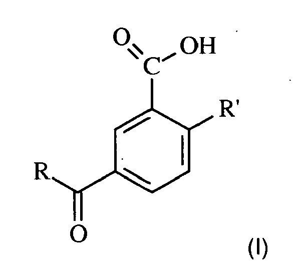 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone; lesdits groupes pouvant être substitués par un ou plusieurs substituants, identiques ou différents, choisis parmi (a) les atomes d'halogène (b) le groupe trifluorométhyle, (c) des groupes hydroxyle sous forme libre ou estérifiée par un acide ayant de 1 à 6 atomes de carbone ou (d) une fonction carboxyle sous forme libre ou estérifiée par un alcool inférieur ayant de 1 à 6 atomes de carbone ;
- R' est un groupe hydroxyle ;
- ainsi que leurs sels issus d'une base minérale ou organique.

2. Composition selon la revendication précédente, **caractérisée par le fait que** l'actif est choisi parmi l'ascorbyl phosphate de magnésium, l'acide ascorbique glycosylé, et leur mélange.

3. Composition selon la revendication 1, **caractérisée par le fait que** l'actif est choisi parmi l'acide salicylique ou l'acide n-octanoyl 5-salicylique.

4. Composition selon l'une quelconque des revendications précédentes, **caractérisée par le fait que** l'actif est présent en une teneur allant de 0,05 % à 10 % en poids, par rapport au poids total de la composition.

5. Composition selon l'une quelconque des revendications précédentes, **caractérisée par le fait que** l'ester d'acide gras et de polyéthylène glycol est choisi parmi les esters d'acide gras en C₁₆-C₂₂ comportant de 8 à 100 unités d'oxyde éthylène.

6. Composition selon l'une quelconque des revendications précédentes, **caractérisée par le fait que** l'ester d'acide gras et de polyéthylène glycol est choisi parmi les stéarates de polyéthylène glycol.

7. Composition selon l'une quelconque des revendications précédentes, **caractérisée par le fait que** l'ester d'acide gras et de polyéthylène glycol comprend de 20 à 40 unités d'oxyde d'éthylène.

8. Composition selon l'une quelconque des revendications précédentes, **caractérisée par le fait qu'**elle comprend un ester d'acide gras en C₁₆-C₂₂ et de sorbitan choisi parmi les stéarates de sorbitan.

9. Composition selon l'une quelconque des revendications précédentes, **caractérisée par le fait qu'**elle comprend un ester d'acide gras en C₁₆-C₂₂ et de glycéryle choisi parmi les stéarates de glycéryle.

10. Composition selon l'une quelconque des revendications précédentes, **caractérisée par le fait qu'**elle comprend un mélange de stéarate de glycéryle et de monostéarate de polyéthylène glycol 100 OE.

11. Composition selon l'une quelconque des revendications précédentes, **caractérisée par le fait qu'**elle comprend un polycondensat tribloc polyéthylène glycol / polypropylène glycol / polyéthylène glycol.

12. Composition selon l'une quelconque des revendications précédentes, **caractérisée par le fait que** le polycondensat d'oxyde d'éthylène et d'oxyde de propylène a un poids moléculaire moyen en poids allant de 1000 à 15000, et de mieux allant de 2000 à 13000.

13. Composition selon la revendication précédente, **caractérisée par le fait qu'**elle comprend gélifiant hydrophile polysaccharide.

14. Composition selon l'une quelconque des revendications précédentes, **caractérisée par le fait qu'**elle comprend au moins un adjuvant cosmétique ou dermatologique choisi parmi les filtres UV, les charges, les conservateurs, les parfums, les bactéricides, les absorbeurs d'odeur, les matières colorantes, les sels, les tensioactifs, les épaississants, les bases.

15. Procédé non thérapeutique de soin ou de maquillage des matières kératiniques comprenant l'application sur les matières kératiniques d'une composition selon l'une quelconque des revendications précédentes.

## Claims

1. Composition in the form of an oil-in-water emulsion comprising:
- a fatty acid and polyethylene glycol ester;
- an additional surfactant chosen from C₁₆-C₂₂ fatty acid and sorbitan esters and C₁₆-C₂₂ fatty acid and glycerol esters;
- an ethylene oxide and propylene oxide polycondensate consisting of polyethylene glycol blocks and polypropylene glycol blocks;
- an active principle chosen from ascorbic acid and its salts, glycosylated ascorbic acid, salicylic acid and salicylic acid derivatives of formula (I): 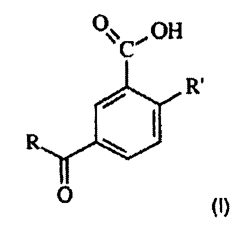 in which:
- the R radical denotes a saturated, linear, branched or cyclic, aliphatic chain having from 2 to 22 carbon atoms; an unsaturated chain having from 2 to 22 carbon atoms and comprising one or more double bonds which may be conjugated; an aromatic ring system bonded to the carbonyl radical directly or via saturated or unsaturated aliphatic chains having from 2 to 7 carbon atoms; it being possible for the said groups to be substituted by one or more identical or different substituents chosen from (a) halogen atoms, (b) the trifluoromethyl group, (c) hydroxyl groups in the free form or in the form esterified by an acid having from 1 to 6 carbon atoms, or (d) a carboxyl functional group in the free form or in the form esterified by a lower alcohol having from 1 to 6 carbon atoms;
- R' is a hydroxyl group;
and their salts resulting from an inorganic or organic base.

2. Composition according to the preceding claim, **characterized in that** the active principle is chosen from magnesium ascorbyl phosphate, glycosylated ascorbic acid and their mixture.

3. Composition according to Claim 1, **characterized in that** the active principle is chosen from salicylic acid or 5-(n-octanoyl)salicylic acid.

4. Composition according to any one of the preceding claims, **characterized in that** the active principle is present in a content ranging from 0.05 to 10% by weight, with respect to the total weight of the composition.

5. Composition according to any one of the preceding claims, **characterized in that** the fatty acid and polyethylene glycol ester is chosen from C₁₆-C₂₂ fatty acid esters comprising from 8 to 100 ethylene oxide units.

6. Composition according to any one of the preceding claims, **characterized in that** the fatty acid and polyethylene glycol ester is chosen from polyethylene glycol stearates.

7. Composition according to any one of the preceding claims, **characterized in that** the fatty acid and polyethylene glycol ester comprises from 20 to 40 ethylene oxide units.

8. Composition according to any one of the preceding claims, **characterized in that** it comprises a C₁₆-C₂₂ fatty acid and sorbitan ester chosen from sorbitan stearates.

9. Composition according to any one of the preceding claims, **characterized in that** it comprises a C₁₆-C₂₂ fatty acid and glycerol ester chosen from glyceryl stearates.

10. Composition according to any one of the preceding claims, **characterized in that** it comprises a mixture of glyceryl stearate and of polyethylene glycol 100 EO monostearate.

11. Composition according to any one of the preceding claims, **characterized in that** it comprises a polyethylene glycol/polypropylene glycol/polyethylene glycol triblock polycondensate.

12. Composition according to any one of the preceding claims, **characterized in that** the ethylene oxide and propylene oxide polycondensate has a weight-average molecular weight ranging from 1000 to 15 000 and better still ranging from 2000 to 13 000.

13. Composition according to the preceding claim, **characterized in that** it comprises a polysaccharide hydrophilic gelling agent.

14. Composition according to any one of the preceding claims, **characterized in that** it comprises at least one cosmetic or dermatological adjuvant chosen from UV screening agents, fillers, preservatives, fragrances, bactericides, odour absorbers, colouring materials, salts, surfactants, thickeners or bases.

15. Nontherapeutic method for caring for or making up keratinous substances comprising the application, to the keratinous substances, of a composition according to any one of the preceding claims.

## Patentansprüche

1. Zusammensetzung in Form einer Öl-in-Wasser-Emulsion, die enthält:
- einen Polyethylenglycolfettsäureester;
- einen ergänzenden grenzflächenaktiven Stoff, der unter den Sorbitanfettsäureestern einer C₁₆-₂₂-Fettsäure und den Glycerinfettsäureestern einer C₁₆-₂₂-Fettsäure ausgewählt ist;
- ein Polykondensat von Ethylenoxid und Propylenoxid, das aus Polyethylenglycolblöcken und Polypropylenglycolblöcken gebildet ist;
- einen Wirkstoff, der unter Ascorbinsäure und ihren Salzen, glycosylierter Ascorbinsäure, Salicylsäure und Salicylsäurederivaten der Formel (I) ausgewählt ist: 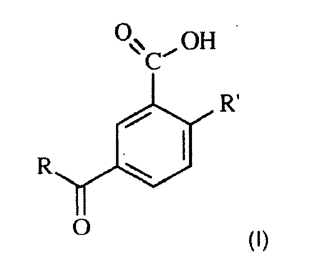 in der Formel:
- die Gruppe R bedeutet eine gesättigte, lineare, verzweigte oder cyclische, aliphatische Kette mit 2 bis 22 Kohlenstoffatomen; eine ungesättigte Kette mit 2 bis 22 Kohlenstoffatomen, die eine oder mehrere Doppelbindungen enthält, die konjugiert sein können; einen aromatischen Ring, der direkt oder über gesättigte oder ungesättigte, aliphatische Ketten mit 2 bis 7 Kohlenstoffatomen an die Carbonylgruppe gebunden sein kann; wobei diese Gruppen mit einem oder mehreren, gleichen oder voneinander verschiedenen Substituenten substituiert sein können, die unter (a) Halogenatomen, (b) der Trifluormethylgruppe, (c) Hydroxygruppen in freier Form oder verestert mit einer Säure mit 1 bis 6 Kohlenstoffatomen oder (d) einer Carboxyfunktion in freier Form oder verestert mit einem niederen Alkohol mit 1 bis 6 Kohlenstoffatomen ausgewählt sind;
- R' ist eine Hydroxygruppe;
sowie ihren mit einer anorganischen oder organischen Base gebildeten Salzen.

2. Zusammensetzung nach dem vorhergehenden Anspruch,
**dadurch gekennzeichnet, dass** der Wirkstoff unter Magnesiumascorbylphosphat, glycosylierter Ascorbinsäure und deren Gemisch ausgewählt ist.

3. Zusammensetzung nach Anspruch 1, **dadurch gekennzeichnet, dass** der Wirkstoff unter Salicylsäure und 5-n-Octanoylsalicylsäure ausgewählt ist.

4. Zusammensetzung nach einem der vorhergehenden Ansprüche, **dadurch gekennzeichnet, dass** der Wirkstoff in einem Mengenanteil im Bereich von 0,05 bis 10 Gew.-%, bezogen auf das Gesamtgewicht der Zusammensetzung, enthalten ist.

5. Zusammensetzung nach einem der vorhergehenden Ansprüche, **dadurch gekennzeichnet, dass** der Polyethylenglycolfettsäureester unter den Estern einer C₁₆₋₂₂-Fettsäure mit 8 bis 100 Ethylenoxideinheiten ausgewählt ist.

6. Zusammensetzung nach einem der vorhergehenden Ansprüche, **dadurch gekennzeichnet, dass** der Polyethylenglycolfettsäureester unter den Polyethylenglycolstearaten ausgewählt ist.

7. Zusammensetzung nach einem der vorhergehenden Ansprüche, **dadurch gekennzeichnet, dass** der Polyethylenglycolfettsäureester 20 bis 40 Ethylenoxideinheiten enthält.

8. Zusammensetzung nach einem der vorhergehenden Ansprüche, **dadurch gekennzeichnet, dass** sie einen Sorbitanfettsäureester einer C₁₆₋₂₂-Fettsäure enthält, der unter den Sorbitanstearaten ausgewählt ist.

9. Zusammensetzung nach einem der vorhergehenden Ansprüche, **dadurch gekennzeichnet, dass** sie einen Glycerinfettsäureester einer C₁₆₋₂₂-Fettsäure enthält, der unter den Glycerylstearaten ausgewählt ist.

10. Zusammensetzung nach einem der vorhergehenden Ansprüche, **dadurch gekennzeichnet, dass** sie ein Gemisch aus Glycerylstearat und Polyethylenglycol(100 EO)monostearat enthält.

11. Zusammensetzung nach einem der vorhergehenden Ansprüche, **dadurch gekennzeichnet, dass** sie ein Dreiblock-Polykondensat Polyethylenglycol/ Polypropylenglycol/ Polyethylenglycol enthält.

12. Zusammensetzung nach einem der vorhergehenden Ansprüche, **dadurch gekennzeichnet, dass** das Polykondensat von Ethylenoxid und Propylenoxid eine gewichtsmittlere Molmasse im Bereich von 1.000 bis 15.000 und noch besser im Bereich von 2.000 bis 13.000 aufweist.

13. Zusammensetzung nach dem vorhergehenden Anspruch,
**dadurch gekennzeichnet, dass** sie ein gelbildendes hydrophiles Polysaccharid enthält.

14. Zusammensetzung nach einem der vorhergehenden Ansprüche, **dadurch gekennzeichnet, dass** sie mindestens einen kosmetischen oder dermatologischen Zusatzstoff enthält, der unter den UV-Filtern, Füllstoffen, Konservierungsmitteln, Parfums, Bakteriziden, Geruchsabsorbern, Farbmitteln, Salzen, grenzflächenaktiven Stoffen, Verdickungsmitteln und Basen ausgewählt ist.

15. Nicht therapeutisches Verfahren zur Pflege und zum Schminken von Keratinsubstanzen, das umfasst, auf die Keratinsubstanzen eine Zusammensetzung nach einem der vorhergehenden Ansprüche aufzutragen.
